# EUROPEAN PATENT SPECIFICATION

(11) **EP 3 573 481 B1**
(45) Date of publication and mention of the grant of the patent: **20.04.2022**
(21) Application number: 18704878.0
(22) Date of filing: 23.01.2018
(51) Int. Cl.: A23L 33/00, A23L 33/20

(54) **DIET COMPOSITION FOR THE PREVENTION AND/OR THE TREATMENT OF ENDOMETRIAL HYPERPLASIA**
DIÄTISCHE ZUSAMMENSETZUNG ZUR PRÄVENTION UND/ODER BEHANDLUNG VON ENDOMETRIUMHYPERPLASIE
COMPOSITION DIÉTÉTIQUE POUR LA PRÉVENTION ET/OU LE TRAITEMENT DE L'HYPERPLASIE ENDOMÉTRIALE

(30) Priority: 26.01.2017 IT 201700008499
(43) Date of publication of application: 04.12.2019
(73) Proprietor: Università degli Studi di Genova, 16126 Genova (IT); L-Nutra Inc., Plano, TX 75024 (US)
(72) Inventor: NENCIONI, Alessio, 16132 Genova (IT); CAFFA, Irene, 17021 Alassio (SV) (IT); BECHERINI, Pamela, 16132 Genova (IT); LONGO, Valter, Culver City, California 90232 (US)
(74) Representative: Ferreccio, Rinaldo
(86) International application number: PCT/EP2018/051590
(87) International publication number: WO 2018/138090

(56) References cited:
- US-A1- 2010 004 309
- US-A1- 2014 227 373
- US-A1- 2016 324 193
- Anonymous: "Science, research and development behind fasting mimicking diet", L-Nutra , 5 December 2016 (2016-12-05), XP002773724, Retrieved from the Internet: URL:https://web.archive.org/web/2016120506 1723/http://l-nutra.com/fasting-mimicking- diet-2/ [retrieved on 2017-09-12]
- Anonymous: "Chemolieve", L-Nutra , 3 January 2017 (2017-01-03), XP002773725, Retrieved from the Internet: URL:https://web.archive.org/web/2017010305 1650/http://l-nutra.com/chemolieve/ [retrieved on 2017-09-12]
- Anonymous: "L-Nutra A Nutri-Technology Company", L-Nutra , 5 November 2016 (2016-11-05), XP002773726, Retrieved from the Internet: URL:https://web.archive.org/web/2016110512 0401/http://l-nutra.com:80/ [retrieved on 2017-09-12]
- DI BIASE STEFANO ET AL: "Fasting-Mimicking Diet Reduces HO-1 to Promote T Cell-Mediated Tumor Cytotoxicity", CANCER CELL, CELL PRESS, US, vol. 30, no. 1, 11 July 2016 (2016-07-11), pages 136-146, XP029636921, ISSN: 1535-6108, DOI: 10.1016/J.CCELL.2016.06.005
- SEBASTIAN BRANDHORST ET AL: "A Periodic Diet that Mimics Fasting Promotes Multi-System Regeneration, Enhanced Cognitive Performance, and Healthspan", CELL METABOLISM, vol. 22, no. 1, 1 July 2015 (2015-07-01), pages 86-99, XP055324968, United States ISSN: 1550-4131, DOI: 10.1016/j.cmet.2015.05.012
- A.S. MCCAMPBELL ET AL: "Loss of p27 Associated with Risk for Endometrial Carcinoma Arising in the Setting of Obesity", CURRENT MOLECULAR MEDICINE, vol. 16, no. 3, 1 March 2016 (2016-03-01), pages 252-265, XP055405477, NL ISSN: 1566-5240, DOI: 10.2174/1566524016666160225153307
- BOUGIE O ET AL: "Influence of Diet on Development of Fibroids and Endometriosis, Analysis of the National Health and Nutrition Examination Survey", JOURNAL OF MINIMALLY INVASIVE GYNECOLOGY, vol. 23, no. 7, 2016, XP029782544, ISSN: 1553-4650, DOI: 10.1016/J.JMIG.2016.08.273
- BERENTSEN V ET AL: "A Diet and Physical Activity Education Program Improves the Symptoms of Endometriosis and Polycystic Ovary Syndrome", JOURNAL OF THE AMERICAN DIETETIC ASSOCIATION, vol. 108, no. 9, 1 September 2008 (2008-09-01), page A118, XP024099668, THE ASSOCIATION, CHICAGO, IL, US ISSN: 0002-8223, DOI: 10.1016/J.JADA.2008.06.363 [retrieved on 2008-08-23]
- MCCANN SUSAN E ET AL: "Diet in the epidemiology of endometrial cancer in Western New York (United States)", CCC. CANCER CAUSES AND CON, vol. 11, no. 10, 1 December 2000 (2000-12-01), pages 965-974, XP009191732, OXFORD, GB ISSN: 0957-5243
- M Mourits: "Tamoxifen treatment and gynecologic side effects: a review", Obstetrics and Gynecology, vol. 97, no. 5, 1 May 2001 (2001-05-01), pages 855-866, XP55694230, US ISSN: 0029-7844, DOI: 10.1016/S0029-7844(00)01196-0
- HOLLOSZY ET AL: "Caloric restriction in humans", EXPERIMENTAL GERONTOLOGY, ELSEVIER, AMSTERDAM, NL, vol. 42, no. 8, 18 July 2007 (2007-07-18), pages 709-712, XP022241800, ISSN: 0531-5565, DOI: 10.1016/J.EXGER.2007.03.009
- DIRKS A J ET AL: "Caloric restriction in humans: Potential pitfalls and health concerns", MECHANISMS OF AGEING AND DEVELOPMENT, ELSEVIER SEQUOIA, LAUSANNE, CH, vol. 127, no. 1, 1 January 2006 (2006-01-01), pages 1-7, XP027961524, ISSN: 0047-6374 [retrieved on 2006-01-01]
- Min Wei ET AL: "Fasting-mimicking diet and markers/risk factors for aging, diabetes, cancer, and cardiovascular disease", Science Translational Medicine, vol. 9, no. 377, 15 February 2017 (2017-02-15), page eaai8700, XP55610354, US ISSN: 1946-6234, DOI: 10.1126/scitranslmed.aai8700

## Description

### Technical Field

The present invention relates to the technical field of the pharmaceutical and dietary industries.

In particular, the invention refers to a diet composition for the prevention and/or the treatment of endometrial hyperplasia in a human subject.

### Prior art

Endometrial hyperplasia is a condition characterized by thickening of the uterine mucosa (endometrium) occuring in response to disproportionate estrogen levels within respect to progesterone ones (https://www.rcog.org.uk/globalassets/documents/guidelines/green-topguidelines/gtg_67_endometrial_hyperplasia.pdf).

Conditions predisposing to endometrial hyperplasia include the use of selective estrogen receptor modulators (ER) such as tamoxifen (a drug used for breast cancer treatment), post-menopausal estrogen intake [hormone replacement therapy (HRT)], obesity, and menstrual irreguralities, especially in women suffering from polycystic ovary syndrome. Endometrial hyperplasia can cause uterine bleeding and predisposes to endometrial cancer development (particularly atypical endometrial hyperplasia) (Kurman et al. Cancer. 1985 Jul 15;56(2):403-12). In women at risk of developing endometrial hyperplasia following use of tamoxifen or HRT, careful clinical or ultrasound scan monitoring is required. In the event of excessive endometrium thickening, interruption of tamoxifen or HRT treatment, invasive gynecological tests and, in some cases, hysterectomy (in the presence of atypical hyperplasia or endometrial carcinoma) may be necessary. In other cases, endometrial hyperplasia, once diagnosed, may require hormone therapy based on oral or topical progestogens. Therefore, it is an extremely common clinical problem with a strong medical, economic and social impact.

Known approaches currently used for the prevention of endometrial hyperplasia
(https://www.rcog.org.uk/globalassets/documents/guidelines/green-topguidelines/gtg_67_endometrial_hyperplasia.pdf) include estrogens and progestogens combination in the post-menopausal replacement therapy.

In women at risk of developing endometrial hyperplasia as a result of overweight or obesity, weight loss by diet or bariatric surgery as well as physical activity can reduce this risk (Campagnoli C, et al. Gynecol Endocrinol. 2013 Feb;29(2):11924; Charalampakis V, et al. Eur J Obstet Gynecol Reprod Biol. 2016 Oct 17). Finally, in women with menstrual disorder, the use of oral contraceptives containing both estrogens and progestogens may be effective in preventing endometrial hyperplasia.

In women taking tamoxifen, the use of levonorgestrel-releasing intrauterine systems (LNG-IUS) reduces the risk of developing endometrial hyperplasia. However, because the effect of the progestogen within LNG-IUS on breast cancer recurrence is unknown, the use of these systems can not be routinely recommended. To the best of the applicants' knowledge, no recommended approaches for the prevention of tamoxifen-induced endometrial hyperplasia are currently available. Therefore, only careful follow-up is possible in women taking this medication.

Known approaches for the treatment of endometrial hyperplasia once it is established (in the absence of evidence of atypical hyperplasia) include the use of oral, parenteral, intrauterine (IUS) progestogens or progestogens administered by vaginal creams. However, the use of progestogens may in turn cause uterine bleeding (https://www.rcog.org.uk/globalassets/documents/guidelines/green-topguidelines/gtg_67_endometrial_hyperplasia.pdf). In cases of atypical endometrial hyperplasia (especially if complex), given the risk of progression to endometrial carcinoma (estimated to be about 22%), hysterectomy is generally recommended.

Previous studies show that periodic fasting or fasting-mimicking diet (FMD) cycles have preventive effects on the toxicity from chemotherapy drugs (DNA damaging agents) (Levine ME, et al. Cell Metab. 2014 Mar 4;19(3):407-17; Raffaghello L, et al. Proc Natl Acad Sci U S A. 2008 Jun 17;105(24):8215-20; US 20130045215 A1, US 8865646 B2, US 9237761 B2, US 20140112909 A1, US 20110118528 A1), but no data regarding the effects of the periodic fasting or the FMD cycles on the side effects of "targeted" drugs used in oncology, such as the tamoxifen-induced endometrial hyperplasia, are available.

It is also known that periodic fasting, FMD, or caloric restriction may reduce risk factors for chronic conditions and possibly lead to a life span extension (Brandhorst S, et al. Cell Metab. 2015 Jul 7;22(1):86-99; Mattison JA, et al. Nature. 2012 Sep 13;489(7415):318-21; Colman RJ, et al. Science. 2009 Jul 10;325(5937):201-4).

It has been suggested that diet, weight loss and caloric/energy restriction (typically defined as a chronic 20-40% reduction of the daily calorie intake with a preserved meal rate) reduce the risk of endometrial disease, including hyperplasia and endometrial cancer (Linkov F, et al. Eur J Cancer. 2008 Aug;44(12):1632-4; Campagnoli C, et al. Gynecol Endocrinol. 2013 Feb;29(2):119-24; Koizumi A, et al. J Nutr. 1990 Nov;120(11):1401-11; McCampbell AS, et al. Curr Mol Med. 2016;16(3):252-65), wherein it is pointed out that caloric/energy restriction is a dietary approach involving several disadvantages and side effects, including weight loss, chronic hunger, reduced wound healing capability and tendency to hypothermia/cold feeling.

It is known from https://web.archive.org/web/20161205061723/http://1-nutra.com/fasting-mimicking-diet-2 / and from https://web.archive.org/web/20170103051650/http://l-nutra.com/chemolieve/ that Fasting Mimicking Diet (FMD) products from L-Nutra, like Prolon and ChemoLieve are to be used 4-5 days per month and have an effect on IGF-1 and may be used to reduce adverse side-effects of chemotherapy and enhance death of cancer cells.

A.S. MCCAMPBELL ET AL: "Loss of p27 Associated with Risk for Endometrial Carcinoma Arising in the Setting of Obesity", Current Molecular Medicine, vol. 16, no. 3, 1 March 2016 (2016-03-01), pages 252-265, and BOUGIE O ET AL: "Influence of Diet on Development of Fibroids and Endometriosis, Analysis of the National Health and Nutrition Examination Survey", Journal of Minimally Invasive Gynecology, vol. 23, no. 7, 2016, disclose that caloric restriction may inhibit growth and decrease development of endometrial hyperplasia.

However, no reliable predictions on the effects that fasting or FMD may have in the prevention and/or treatment of endometrial hyperplasia, particularly endometrial hyperplasia developed following the use of tamoxifen or other selective estrogen receptor modulators (SERMs), can be made.

For these reasons, the research carried out by the Applicants focused on the study of the possible favorable effect of fasting or FMD effect in the prevention and/or treatment of endometrial hyperplasia.

The present invention is the result of the above research activity.

### Summary of the Invention

In one aspect, the present invention refers to a diet composition for use in the prevention and/or the treatment of endometrial hyperplasia in a human subject, the diet composition comprising:
a fasting mimicking diet component to be administered for a first time period, said fasting mimicking diet component containing complex carbohydrates from plant sources and providing less than 50% of the normal caloric intake of the subject with both protein restriction and sugar restriction, as it provides the subject with no more than 0.4 g proteins/kg of body weight/day and no more than 11 kcal/kg of bodyweight/day and contains an amount of sugars that provides less than 15% of the total calories provided by the fasting mimicking diet component; and
a re-feeding diet component to be administered for a second time period, said re-feeding diet component providing 60-100% of the normal caloric intake of the subject;
wherein the fasting mimicking diet component and the re-feeding diet component are administered for multiple cycles.

Preferably, the first time period is from 2 to 10 days and the second time period is from 7 to 85 days.

More preferably, the first time period is 5 days, and the second time period is 25-26 days.

Preferably, the multiple cycles comprise one administration once a month for at least 2 months.

Examples of FMD protocols that can be used in the present invention are found in patent applications US 2015/0133370 A1 and US 2014/0328863 A1.

The fasting mimicking diet component provides the subject preferably with no more than 1160 kcal/day, and in particular, no more than 800 kcal/day.

In one embodiment, the fasting mimicking diet component provides the subject with 100 to 1000 kcal/day.

The fasting mimicking diet component provides the subject, with 1000, 957, 700, 500, 300, or 100 kcal/day, in ascending order of preference.

In one embodiment, the fasting mimicking diet component provides the subject with a protein amount less than or equal to 36 g/day. In particular, the fasting mimicking diet component provides the subject with a protein amount equal to 36, 20, 10, or 5 or 0 g/day, in increasing order of preference.

Preferably the fasting mimicking diet component provides the subject with 2-8 kcal/kg of body weight/day.

Preferably the fasting mimicking diet component provides the subject with no more than 0.3, 0.2 or 0.1 g proteins/kg of body weight/day.

Other examples of FMD can be found in the WO 2014/066426 and WO 2014/127000 applications.

Lists of nutrients contained in the fasting mimicking diet component referring to a 80-90 kg subject, are shown in Tables 1-2 below.

**Table 1**

| | Day 1 | Days 2,3,4,5 |
|---|---|---|
| Total calories | 1152 | 809 |
| Fats | 56% | 46% |
| Carbohydrates | 34% | 46% |
| of which sugars | 10% | 9% |
| Protein | 10% | 9% |

**Table 2**

| | Unit | Day 1 | %DD | Days 2,3,4,5 | %DD | mean %DD |
|---|---|---|---|---|---|---|
| Protein | g | 29 | | 18 | | |
| Fats | g | 72 | | 41 | | |
| Carbohydrates (by difference) | g | 98 | | 91 | | |
| From sugars | g | 29 | | 17.6 | | |
| Dietary fiber | g | 22 | 86 | 14 | 56 | 62 |
| Calcium | mg | 604 | 60 | 426 | 43 | 46 |
| Iron | mg | 13 | 77 | 10 | 55 | 60 |
| Magnesium | mg | 387 | 97 | 230 | 58 | 65 |
| Phosphorus | mg | 390 | 39 | 276 | 28 | 30 |
| Potassium | mg | 2519 | 72 | 1795 | 51 | 55 |
| Sodium | mg | 2427 | 101 | 1750 | 73 | 79 |
| Zinc | mg | 7 | 46 | 4.2 | 28 | 32 |
| Copper | mg | 1.5 | 76 | 1.2 | 59 | 63 |
| Manganese | mg | 3 | 148 | 1.9 | 95 | 105 |
| Selenium | mg | 7 | 10 | 5.3 | 8 | 8 |
| Vit. A | IU | 39254 | 785 | 27549 | 551 | 598 |
| Vit. C | mcg | 236 | 393 | 138 | 229 | 261 |
| Vit. B1 | mg | 4 | 209 | 2.2 | 113 | 132 |
| Vit. B2 | mg | 3.8 | 191 | 2 | 109 | 126 |
| Vit. B3 (niacin) | mg | 28.5 | 143 | 18 | 92 | 102 |
| Vit. B5 (pantothenic acid) | mg | 1.2 | 12 | 1.0 | 10 | 10 |
| Vit. B6 | mg | 4.0 | 200 | 2.2 | 111 | 129 |
| Vit. B9 (folate) | mg | 479 | 120 | 317 | 79 | 87 |
| Vit. B12 | mcg | 16 | 227 | 16 | 227 | 227 |
| Vit. D | IU | 952 | 238 | 952 | 238 | 238 |
| Vit. E | mcg | 25 | 127 | 16 | 80 | 89 |
| Vit. K | mg | 1795 | 2243 | 1110 | 1387 | 1559 |

| | | | | | | |
|---|---|---|---|---|---|---|
| DD = Daily dose | | | | | | |

Typically, in the FMD protocol the usual diet of the subjects is substituted for a predefined number of days (e.g. 5 days), in which the subject drinks plenty of water. For normal weight subjects (Body Mass Index between 18.5 and 25), the fasting mimicking diet component is taken up once a month (preferably for 5 days) while for the next 25-26 days the subject receives the re-feeding diet component.

This is for the first 3 months; subsequently the subject receives the fasting mimicking diet component for 5 days and the re-feeding diet component for about 85 days continuing with a 5-day cycle of fasting mimicking diet component followed by 85 days of re-feeding diet component. The subject weight is monitored so that the subject reacquires at least 95% of the weight lost during the administration of the fasting mimicking diet component, before starting the new cycle (for normal weight subjects). In overweight subjects, a weight loss following FMD cycles is admissible as long as it is well tolerated and the weight of the subject does not drop below the normal BMI range.

Preferably, the fasting mimicking diet component comprises proteins in an amount that is less than 15% of the total calories provided by the fasting mimicking diet component.

Preferably, the fasting mimicking diet component comprises sugars in an amount that is less than 15% of the total calories provided by the fasting mimicking diet component.

Preferably, the fasting mimicking diet component provides the subject with 6 to 10 kcal/kg of body weight/day on days 2 to 5.

Most preferably, the fasting mimicking diet component provides the subject with 4 to 8 kcal/kg/body weight/day on days 2 to 5.

Preferably, the fasting mimicking diet component comprises at least 60% calories from fatty acids, 2-5% calories from glycerol and up to 5% calories from plant-based proteins and a maximum of 35% calories from carbohydrates.

Preferably, said fasting mimicking diet component comprises complex carbohydrates from plant sources, which preferably comprise soy, rice or other cereals.

Preferably at least 50% of the calories from fatty acids are from coconut oil and tree nuts. The latter preferably comprise walnuts, macadamia nuts and/or almonds.

Then the subject is fed with food with a high content of monounsaturated and polyunsaturated fats and a reduced content of proteins and sugars (≥ 40% calories coming from fat). This is because a diet based on these foods has beneficial effects that are similar to those of fasting [13].

It is also described a method for the prevention and the treatment of endometrial hyperplasia in a human patient, wherein the method comprises subjecting said patient to a reduced caloric intake over a period of 24-190 hours.

By reduced caloric intake it is herein meant a daily caloric intake reduced by 10-100%, preferably 50-100%, more preferably 75-100%, with respect to the regular caloric intake, including total fasting.

The regular caloric intake of the subject is the number of kcal that the subject consumes in order to maintain its weight. The normal caloric intake of the subject can be estimated by interviewing the subject or by considering the subject weight. As a rough guide, the normal caloric intake of the subject is on average 2600 kcal/day for men and 1850 kcal/day for women.

Preferably, when the daily caloric intake is reduced by 10-85%, the patient is fed with foods with a high content of monounsaturated and polyunsaturated fats and a reduced content of proteins and sugars (≥ 40% calories coming from fat). This is because a diet based on these foods has beneficial effects that are similar to those of fasting [13].

Preferably, said reduced caloric intake period ranges from 48 to 150 hours, and more preferably is about 120 hours.

The above mentioned reduced caloric intake period can be repeated one or more times after respective periods of 5-180 days, during which the patient follows a diet involving a regular caloric intake.

The aforementioned reduced caloric intake regime preferably corresponds to less than 800 kcal/day, more preferably 400 kcal/day.

The present invention will be further described with reference to the enclosed drawings and to some eembodiments, which are provided below for illustrative and non-limiting purposes.

### Brief description of the drawings

Figure 1A is a histogram showing the weight of the uterus of BALB/c mice, wherein the mice were subjected to normal diet, tamoxifen treatment and normal diet, fasting (water only), or tamoxifen treatment and fasting, respectively.
Figure 1B is a histogram showing the weight of the uterus of BALB/c mice, wherein the mice were subjected to normal diet, tamoxifen treatment and normal diet, an FMD (ChemoLieve^{™}) or tamoxifen treatment + FMD (ChemoLieve^{™}).
Figure 2 shows pictures of the uterus taken from the above BALB/c mice, subjected to the treatments set out in Figures 1A and 1B.
Figure 3 presents pictures from histological analyses carried out on the uteri set out in Figure 2.
Figure 4 is a diagram showing the *Igfr1* mRNA quantification in the uteri of mice subjected to the treatments set out in Figures 1A and 1B.
Figure 5 is a diagram showing *Tff1* (an estrogen receptor target gene, ER) mRNA quantification in the uteri of mice subjected to the treatments set out in Figures 1A and 1B.

### Detailed Description

The invention relates to a diet composition as described above for the prevention and/or the treatment of endometrial hyperplasia. The diet composition is administered as described above to women at risk of developing endometrial hyperplasia (following HRT or tamoxifen intake, in the context of obesity or menstrual irregularities - in the latter case, there is the indication for use of the diet composition particularly when the menstrual irregularities reflect a polycystic ovary syndrome) or with previously diagnosed endometrial hyperplasia. It is specified that the use of this diet composition does not exclude hysterectomy, in cases in which the presence of atypical hyperplasia requires it.

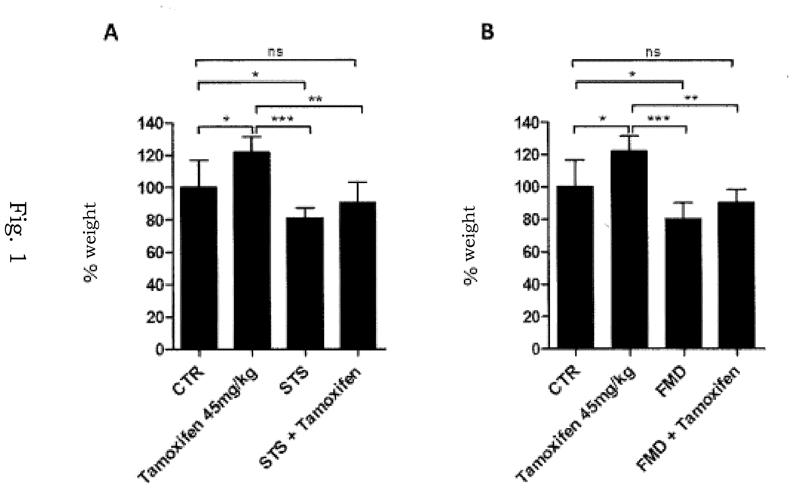

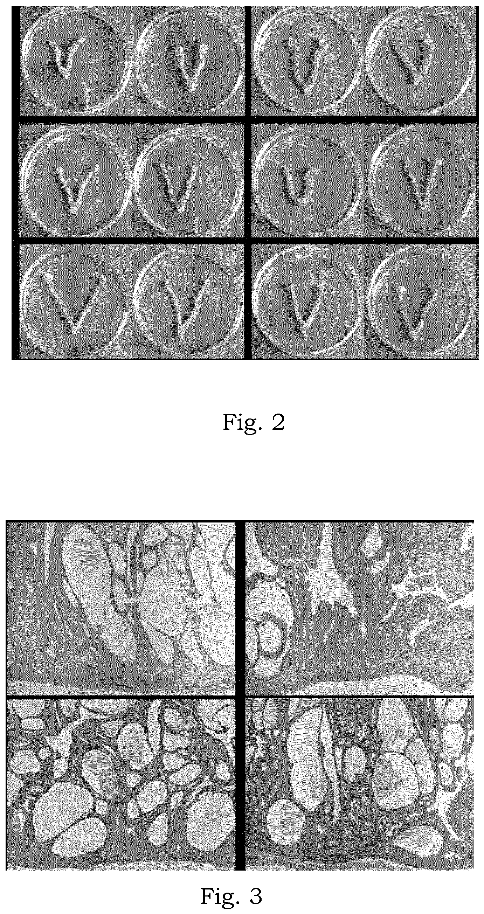

The finding that animals treated for four weeks with tamoxifen (a drug which induces endometrial hyperplasia acting as a partial ER agonist at uterus level) show an increase in uterus weight (see Figures 1A and B), an increase in the size of the uterus itself (Figure 2) and a histological pattern of uterine hyperplasia (Figure 3), supports the importance of the invention. However, mice subjected to fasting cycles (only water for 48h) or an FMD (ChemoLieve^{™} of L-Nutra) and concurrent tamoxifen treatment did not develop any of these effects (Figures 1-3).

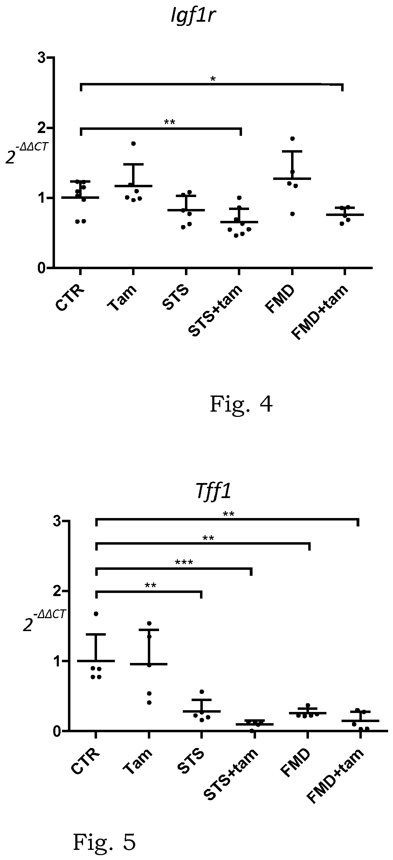

In particular, in the experiments set out in Figures 1-5, 6-8 weeks old BALB/c mice were used, which were randomly distributed to one of six groups (five mice per treatment group): control (normal diet); tamoxifen (normal diet with tamoxifen, given at a dosage of 30 mg/kg/day by gastric gavage); fasting (only water for 48 h once a week); FMD (ChemoLieve^{™}; 72 h once a week); fasting + tamoxifen; FMD + tamoxifen. After five weeks of treatment, the mice were sacrificed and the taken uteri were weighed (Fig. 1A and 1B), photographed (Fig. 2), fixed for subsequent histological analysis (Fig. 3), or subjected to flash freezing and subsequently used for the RNA extraction and the *Igfr1* and *Tff1* mRNA quantification (Fig. 4, 5).

Both fasting and fasting mimicking diet (FMD) were found to have reduced uterus weight and prevented the tamoxifen-induced uterus weight increase (Fig. 1, 2). Prevention of tamoxifen-induced endometrial hyperplasia by fasting was promptly confirmed by histology (Fig. 3).

Previous studies suggested a role for the Igf-1 growth factor-activated signaling cascade in making endometrial cells sensitive to the mitogenic effects of estrogens or, plausibly, of tamoxifen (which acts primarily as a partial estrogen receptor agonist in endometrium) (Eritja N, Mirantes C, Llobet D, Yeramian A, Bergada L, Dosil MA, Domingo M, Matias-Guiu X, Dolcet X: "Long-term estradiol exposure is a direct mitogen for insulin/EGF-primed endometrial cells and drives PTEN loss-induced hyperplasic growth". The American Journal of pathology 2013, 183(1):277-287).

It is interesting to note that, in the experiments conducted, an Igf-1 receptor mRNA downregulation (*Igf1R*) was detected in the uteri of mice which were fasted or subjected to FMD during tamoxifen treatment (Fig. 4). This suggests that fasting may downregulate the Igf-1-induced signaling and that this effect may be one of the mechanisms by which fasting itself or FMD protect against tamoxifen-induced endometrial hyperplasia. In response to fasting or FMD used alone or in combination with tamoxifen, very low levels of *Tff1*mRNA were also measured in the uteri of mice and this indicates that the estrogen receptor (ER) activity, which precisely controls *Tff1* expression, is blocked by fasting or FMD. This aspect, i.e. blocking the ER function by fasting or FMD, could represent a further mechanism underlying the countering of endometrial hyperplasia by these dietary measures.

Both fasting and FMD cycles were well tolerated, causing a transient weight loss that was subsequently readily recovered by the animals between cycles.

Overall, the above experimental results clearly demonstrate the capability of fasting or FMD to prevent or treat endometrial hyperplasia.

The main advantages of the invention lie in the possibility of preventing a side effect, which is annoying and heavily affecting the quality of life of women undergoing tamoxifen or HRT therapy. The onset of uterine hyperplasia may in fact lead to bleeding and, even when it is asymptomatic, involves the burden of careful clinical or ultrasound monitoring and sometimes invasive biopsy investigations.

Periodic cycles of a FMD with the diet composition according to the invention during tamoxifen therapy or HRT, or when obesity or menstrual irregularities are present in the context of a polycystic ovarian syndrome might represent a sufficient approach for preventing uterine hyperplasia thus significantly improving the quality of life of patients as well as reducing the risk of developing endometrial cancer.

Compared to the use of caloric restriction for the prevention or the treatment of endometrial hyperplasia, the use of FMD cycles would have the advantage of allowing a normal diet between cycles and avoiding the side effects of caloric restriction itself (e.g. weight loss, hunger, hypothermia, reduced wounds healing capability). Prevention of uterine hyperplasia by FMD in women taking tamoxifen would also allow not to interrupt the tamoxifen therapy, thus continuing to benefit from the antineoplastic activity of this drug for the entire period of time in which the intake thereof is recommended. In these women, this would have the additional advantage of increasing the antineoplastic activity of tamoxifen itself, which would likely result in an improvement of the prognosis of the patients themselves (in this regard see Italian Patent Application No. 102016000017036 of the same Applicants).

In the cases of women diagnosed with uterine hyperplasia (i.e. in the presence of an already developed uterine hyperplasia), FMD cycles with the diet composition according to the invention could be prescribed with therapeutic purpose, allowing to avoid the topical or systemic use of progestogens and the adverse effects that these drugs can cause (e.g. bleeding).

## Claims

1. A diet composition for use in the prevention and/or the treatment of endometrial hyperplasia in a human subject, the diet composition comprising:
- a fasting mimicking diet component to be administered for a first time period, said fasting mimicking diet component containing complex carbohydrates from plant sources and providing less than 50% of the normal caloric intake of the subject with both protein restriction and sugar restriction, as it provides the subject with no more than 0.4 g proteins/kg of body weight/day and no more than 11 kcal/kg of bodyweight/day and contains an amount of sugars that provides less than 15% of the total calories provided by the fasting mimicking diet component; and
- a re-feeding diet component to be administered for a second time period, said re-feeding diet component providing 60-100% of the normal caloric intake of the subject;
wherein the fasting mimicking diet component and the re-feeding diet component are administered for multiple cycles.

2. The diet composition of claim 1, wherein said first time period is from 2 days to 10 days, preferably 5 days, and said second time period is from 7 to 85 days, preferably 25-26 days.

3. The diet composition according to claim 1 or 2, wherein said multiple cycles comprise an administration once a month for at least 2 months.

4. The diet composition according to any one of claims 1-3, wherein said fasting mimicking diet component provides the subject with no more than 1160 kcal/day, preferably 100-1000 kcal/day.

5. The diet composition according to any one of claims 1-4, wherein said fasting mimicking diet component provides the subject with a protein amount of 0-20 g/day.

6. The diet composition according to any one of claims 1-5, wherein said fasting mimicking diet component comprises carbohydrates in such an amount as to provide no more than half of the calories provided by said diet component.

7. The diet composition according to any one of claims 1-6, wherein said fasting mimicking diet component provides the subject with 2-8 kcal/kg of body weight/day.

8. The diet composition according to claim 7, wherein said fasting mimicking diet component provides the subject with proteins in an amount that is 0.1-0.3 g/kg of body weight/day.

9. The diet composition according to any one of claims 1-8, wherein said fasting mimicking diet component comprises proteins in an amount that is less than 15% of the total calories provided by the fasting mimicking diet component.

10. The diet composition according to any one of claims 2-9, wherein said first time period is 5 days and said fasting mimicking diet component provides the subject with 6 to 10 kcal/kg of body weight/day (preferably 4-8 kcal/kg of body weight/day) for days 2 to 5.

11. The diet composition according to any one of claims 1-10, wherein said fasting mimicking diet component comprises at least 60% calories from fatty acids, 2-5% calories from glycerol and up to 5% calories from plant-based proteins and a maximum of 35% calories from carbohydrates.

12. The diet composition according to claim 11, wherein said fasting mimicking diet component comprises complex carbohydrates from soy, rice or other grains.

13. The diet composition according to claim 11 or 12, wherein at least 50% of the calories from fatty acids are from coconut oil and tree nuts, which preferably comprise walnuts, macadamia nuts and/or almonds.

## Patentansprüche

1. Diätzusammensetzung zur Verwendung bei der Prävention und/oder der Behandlung von Endometriumhyperplasie bei einem Menschen, wobei die Diätzusammensetzung Folgendes aufweist:
- eine Fasten nachahmende Diätkomponente, die für einen ersten Zeitraum zu verabreichen ist, wobei die Fasten nachahmende Diätkomponente komplexe Kohlenhydrate aus pflanzlichen Quellen enthält und weniger als 50 % der normalen Kalorienaufnahme der Person mit sowohl Proteineinschränkung als auch Zuckereinschränkung bereitstellt, indem sie die Person mit nicht mehr als 0,4 g Proteine/kg Körpergewicht/Tag und nicht mehr als 11 kcal/kg Körpergewicht/Tag versorgt und eine Menge an Zuckern enthält, die weniger als 15 % der Gesamtkalorien ausmacht, die von der Fasten nachahmenden Diätkomponente bereitgestellt werden; und
- eine Ernährungswiederaufbau-Diätkomponente, die für einen zweiten Zeitraum zu verabreichen ist, wobei die Ernährungswiederaufbau-Diätkomponente 60 -100 % der normalen Kalorienaufnahme der Person bereitstellt; wobei die das Fasten nachahmende Diätkomponente und die Nahrungswiederaufbau-Diätkomponente für mehrere Zyklen verabreicht werden.

2. Diätzusammensetzung nach Anspruch 1,
wobei der erste Zeitraum von 2 Tagen bis 10 Tagen, vorzugsweise 5 Tage, beträgt und der zweite Zeitraum von 7 bis 85 Tagen, vorzugsweise 25-26 Tage, beträgt.

3. Diätzusammensetzung nach Anspruch 1 oder 2,
wobei die mehreren Zyklen eine Verabreichung einmal im Monat für mindestens 2 Monate umfassen.

4. Diätzusammensetzung nach einem der Ansprüche 1 bis 3,
wobei die Fasten nachahmende Diätkomponente die Person mit nicht mehr als 1160 kcal/Tag, vorzugsweise 100-1000 kcal/Tag, versorgt.

5. Diätzusammensetzung nach einem der Ansprüche 1 bis 4,
wobei die Fasten nachahmende Diätkomponente die Person mit einer Proteinmenge von 0 bis 20 g/Tag versorgt.

6. Diätzusammensetzung nach einem der Ansprüche 1 bis 5,
wobei die Fasten nachahmende Diätkomponente Kohlenhydrate in einer solchen Menge aufweist, dass sie nicht mehr als die Hälfte der von der Diätkomponente bereitgestellten Kalorien liefert.

7. Diätzusammensetzung nach einem der Ansprüche 1 bis 6,
wobei die Fasten nachahmende Diätkomponente die Person mit 2-8 kcal/kg Körpergewicht/Tag versorgt.

8. Diätzusammensetzung nach Anspruch 7,
wobei die Fasten nachahmende Diätkomponente die Person mit Proteinen in einer Menge versorgt, die 0,1 - 0,3 g/kg Körpergewicht/Tag beträgt.

9. Diätzusammensetzung nach einem der Ansprüche 1 bis 8,
wobei die Fasten nachahmende Diätkomponente Proteine in einer Menge aufweist, die weniger als 15 % der Gesamtkalorien beträgt, die von der Fasten nachahmenden Diätkomponente bereitgestellt werden.

10. Diätzusammensetzung nach einem der Ansprüche 2 bis 9,
wobei der erste Zeitraum 5 Tage beträgt und die Fasten nachahmende Diätkomponente die Person mit 6 bis 10 kcal/kg Körpergewicht/Tag (vorzugsweise 4-8 kcal/kg Körpergewicht/Tag) an den Tagen 2 bis 5 versorgt.

11. Diätzusammensetzung nach einem der Ansprüche 1 bis 10,
wobei die Fasten nachahmende Diätkomponente mindestens 60 % Kalorien aus Fettsäuren, 2-5 % Kalorien aus Glycerin und bis zu 5 % Kalorien aus Proteinen auf Pflanzenbasis und maximal 35 % Kalorien aus Kohlenhydraten aufweist.

12. Diätzusammensetzung nach Anspruch 11,
wobei die Fasten nachahmende Diätkomponente komplexe Kohlenhydrate aus Soja, Reis oder anderen Getreiden aufweist.

13. Diätzusammensetzung nach Anspruch 11 oder 12,
wobei mindestens 50 % der Kalorien aus Fettsäuren aus Kokosnussöl und Baumnüssen stammen, die vorzugsweise Walnüsse, Macadamianüsse und/ oder Mandeln umfassen.

## Revendications

1. Composition de régime destinée à être utilisée dans la prévention et/ou le traitement de l'hyperplasie de l'endomètre chez un sujet humain, la composition de régime comprenant :
- un composant de régime imitant le jeûne à administrer pendant une première période de temps, ledit composant de régime imitant le jeûne contenant des glucides complexes provenant de sources végétales et fournissant moins de 50 % de l'apport calorique normal du sujet avec à la fois une restriction de protéine et une restriction de sucre, étant donné qu'il fournit au sujet pas plus de 0,4 g de protéines/kg de poids corporel/jour et pas plus de 11 kcal/kg de poids corporel/jour et contient une quantité de sucres qui fournit moins de 15 % des calories totales fournies par le composant de régime imitant le jeûne ; et
- un composant de régime de réalimentation à administrer pendant une seconde période de temps, ledit composant de régime de réalimentation fournissant 60 à 100 % de l'apport calorique normal du sujet ;
dans laquelle le composant de régime imitant le jeûne et le composant de régime de réalimentation sont administrés pour plusieurs cycles.

2. Composition de régime selon la revendication 1, dans laquelle ladite première période de temps est de 2 jours à 10 jours, de préférence de 5 jours, et ladite seconde période de temps est de 7 à 85 jours, de préférence de 25 à 26 jours.

3. Composition de régime selon la revendication 1 ou 2, dans laquelle lesdits cycles multiples comprennent une administration une fois par mois pendant au moins 2 mois.

4. Composition de régime selon l'une quelconque des revendications 1 à 3, dans laquelle ledit composant de régime imitant le jeûne fournit au sujet pas plus de 1160 Kcal/jour, de préférence de 100 à 1000 kcal/jour.

5. Composition de régime selon l'une quelconque des revendications 1 à 4, dans laquelle ledit composant de régime imitant le jeûne fournit au sujet une quantité de protéine de 0 à 20 g/jour.

6. Composition de régime selon l'une quelconque des revendications 1 à 5, dans laquelle ledit composant de régime imitant le jeûne comprend des glucides en une quantité suffisante pour ne pas fournir plus de la moitié des calories fournies par ledit composant de régime.

7. Composition de régime selon l'une quelconque des revendications 1 à 6, dans laquelle ledit composant de régime imitant le jeûne fournit au sujet 2 à 8 kcal/kg de poids corporel/jour.

8. Composition de régime selon la revendication 7, dans laquelle ledit composant de régime imitant le jeûne fournit au sujet des protéines en une quantité qui est de 0,1 à 0,3 g/kg de poids corporel/jour.

9. Composition de régime selon l'une quelconque des revendications 1 à 8, dans laquelle ledit composant de régime imitant le jeûne comprend des protéines en une quantité qui est inférieure à 15 % des calories totales fournies par le composant de régime imitant le jeûne.

10. Composition de régime selon l'une quelconque des revendications 2 à 9, dans laquelle ladite première période de temps est de 5 jours et ledit composant de régime imitant le jeûne fournit au sujet 6 à 10 kcal/kg de poids corporel/jour (de préférence 4 à 8 kcal/kg de poids corporel/jour) pendant les jours 2 à 5.

11. Composition de régime selon l'une quelconque des revendications 1 à 10, dans laquelle ledit composant de régime imitant le jeûne comprend au moins 60 % de calories provenant d'acides gras, 2 à 5 % de calories provenant de glycérol et jusqu'à 5 % de calories provenant de protéines d'origine végétale et un maximum de 35 % de calories provenant de glucides.

12. Composition de régime selon la revendication 11, dans laquelle ledit composant de régime imitant le jeûne comprend des glucides complexes provenant du soja, du riz ou d'autres céréales.

13. Composition de régime selon la revendication 11 ou 12, dans laquelle au moins 50 % des calories provenant des acides gras proviennent de l'huile de noix de coco et des fruits à coque, qui comprennent de préférence des noix, des noix de macadamia et/ou des amandes.
